# EUROPEAN PATENT SPECIFICATION

(11) **EP 2 817 045 B1**
(45) Date of publication and mention of the grant of the patent: **27.01.2016**
(21) Application number: 13702468.3
(22) Date of filing: 01.02.2013
(51) Int. Cl.: A61M 5/20, A61M 5/48, A61M 5/50, A61M 5/315

(54) **AN END OF DOSE INDICATOR**
ENDE EINER DOSISANZEIGE
EXTRÉMITÉ D'INDICATEUR DE DOSE

(30) Priority: 21.02.2012 EP 12156376; 28.02.2012 US 201261604137 P
(43) Date of publication of application: 31.12.2014
(73) Proprietor: Novo Nordisk A/S, 2880 Bagsværd (DK)
(72) Inventor: KIILERICH, Ebbe, 2800 Bagsværd (DK)
(86) International application number: PCT/EP2013/052086
(87) International publication number: WO 2013/124139

(56) References cited:
- EP-A1- 0 594 357
- EP-A2- 0 730 876
- WO-A1-2010/035059
- WO-A1-2011/003980
- US-A1- 2010 137 792

## Description

### THE TECHNICAL FIELD OF THE INVENTION:

The present invention relates to a drug delivery device having means for providing signals to the user, especially signals indicating to the user that liquid drug has stopped flowing from the injection device. More specifically the invention relates to an injection device having a mechanical pressure indicator which provides a signal to a user once the pressure inside a container drops below a certain threshold value set such that liquid does not flow from the container.

### DESCRIPTION OF RELATED ART:

People suffering from diabetes have to inject themselves with insulin at a daily basis. For this purpose a great number of different injection systems have been developed over the last 30 years.

A medical delivery apparatus as defined in the preamble of claim 1 is disclosed in US 2010/0137792.

The typical diabetes patient will require injections of insulin several times during the course of a week or a day. However, typical injection devices do not address the problem of indicating when the insulin has been properly delivered into the body.

When performing an injection, the user dials the size (usually the volume) of the intended dose where after he inserts the injection needle into a suitable region of his body and activates the injection button.

In some injection devices, the force needed to press the insulin out from the container is delivered by the user himself (e.g. US 6,004,297), in some devices the force is delivered from a spring (e.g. EP 1,819,382) and in other injection devices the force arises from an electric motor (e.g. EP 1,239,902). No matter how the required force is provided, it takes some time to physically press out the set dose from the cartridge, and it takes some time for the volume of the dose to flow through the needle and into the body. Further, since the plunger pressing the liquid drug from the container is usually made from a rubber material it will be deformed when pressed forward, and it will take some time after the plunger has been forced forward before it reflects back to its original shape. During this time the plunger urges a forward force on the liquid drug, as the plunger is usually prevented from settling backwards due to the presence of the piston rod. During this time the injection needle should stay properly inserted into the body in order for all of the drug to be delivered into the body. If the injection needle is removed from the body to soon some quantity of the set dose will be delivered outside the body.

For most injection devices, the manufacture recommend to keep the needle inserted approximately 5-10 seconds after the injection has been performed. Such recommended time interval is based on calculations involving an "average user". However, it requires the user to either count the seconds or to have access to a stop watch to keep track of the time in order to follow the recommendation.

In order to signal to the user that the injection needle can be removed from the body it has been suggested in EP 1,017,435 to provide a time delay mechanism which is activated during injection and which mechanism provides the user with an acoustic or vibration signal a predetermined time interval after the actual injection has been performed.

However, using a predetermined time interval, or a predetermined recommendation, will almost for certain result in either too short a time interval or too long a time interval as the time required depend on the volume of the dose. It would thus be recommendable to have the time interval the injection needle should stay inserted depend on the size of the dose injected.

Regarding pressure surveillance, US 4,624,659 disclose an injection device provided with a membrane which is in communication with the interior of the syringe barrel through a bore. When a pressure is produced in the barrel, this pressure is transmitted to the backside of the membrane and makes the membrane bulge upward. The bulging of the membrane provides a tactile and visual signal to the user that the pressure in the barrel is above a certain value. The purpose of this is to guide the user to inject at an adequate injection pressure.

### DESCRIPTION OF THE INVENTION:

Having regard to the above identified prior art, it is an object of the present invention to provide a user with a more precise signal indicating when the injection needle can be removed from the body. The signal provided is preferably generated solely by mechanical means making it suitable for a non-electrical injection device. It is further an object to provide such signal in dependency of the size of the dose injected. Yet, a further object is to provide a signal which indicates that the pressure inside the container containing the liquid drug is below a certain threshold value. A threshold value set at a level indicating that no more liquid flow through the injection needle.

The invention is defined in claim 1.

In a first aspect, the present invention relates to an injection mechanism comprising a resilient member such as a spring. This resilient member is capable of operating between two states;
- a relaxed state in which the resilient member is relaxed, and
- a tensed state in which the resilient member is tensed.

When a user operates the injection mechanism to expel a dose, a pressure is build up in the hollow drug container of the injection mechanism. As pressure is being build up i.e. during injection into the tissue of a patient, the resilient member is moved from its relaxed state to its tensed state. Further, means are provided to maintain the resilient member in its tensed state as long as the pressure inside the liquid drug compartment is above a certain threshold value.

As the liquid drug flow out through the lumen of an injection needle connected to the injection mechanism, the pressure inside the drug container falls until the inside pressure balances of the outside pressure. If injection is performed into a patient, the outside pressure is that of the pressure in the tissue of the patient being injected.

Once the pressure has dropped below the threshold value, the resilient member is released and automatically moves back to its relaxed state. This movement of the resilient member occurs rather abrupt whereby an audible and/or tactile signal is provided to the user. This signal informs the user that the inside pressure in the drug container is at a level where the liquid drug has stopped to flow from the drug container or at least that the amount flowing is very little, thus the user can safely remove the injection needle from the tissue.

The injection mechanism contemplated by the present invention is a traditional injection mechanism in which a piston rod moves a plunger forward inside a tubular cartridge in order to press out the liquid drug contained in the cartridge. When the plunger is moved forward inside the cartridge and a pressure is being build up, this pressure applies an axial force on the plunger and on the piston rod, i.e. the pressure in the drug container applies an opposite directed axial force to the piston rod and to the piston rod system. The core of the invention lies in using this axial force to secure the resilient member in its tensed state.

The resilient member being an element which can be operated from a relaxed state to a tensed state and further inherently has the capability of changing from its tensed state and back to its relaxed state when no, or only little, force or pressure is applied to the resilient element.

The locking of the resilient member in the tensed state can in one example be done by having a radial movable gear wheel being blocked in its radial movement by a frictional force occurring between the gear wheel and a toothed piston rod.

In a further example an axial movable nut member can be used to secure the resilient member. This axial movable nut member is pressed to its securing position by the pressure inside the liquid drug compartment.

In the latter example it is preferred to use a plate spring which has a larger outside diameter in its tensed position which larger diameter can be used to be jammed by the axial movable nut member.

### DEFINITIONS:

An "injection pen" is typically an injection apparatus having an oblong or elongated shape somewhat like a pen for writing. Although such pens usually have a tubular cross-section, they could easily have a different cross-section such as triangular, rectangular or square or any variation around these geometries.

As used herein, the term **"drug"** is meant to encompass any drug-containing flowable medicine capable of being passed through a delivery means such as a hollow needle in a controlled manner, such as a liquid, solution, gel or fine suspension. Representative drugs includes pharmaceuticals such as peptides, proteins (e.g. insulin, insulin analogues and C-peptide), and hormones, biologically derived or active agents, hormonal and gene based agents, nutritional formulas and other substances in both solid (dispensed) or liquid form.

Correspondingly, the term **"subcutaneous"** injection is meant to encompass any method of transcutaneous delivery to a subject.

Further the term **"injection needle"** defines a piercing member adapted to penetrate the skin of a subject for the purpose of delivering or removing a liquid.

The term **"Needle Cannula"** is used to describe the actual conduit performing the penetration of the skin during injection. A needle cannula is usually made from a metallic material and connected to a "hub" to form an injection needle. A needle cannula could however also be made from a polymeric material or a glass material. The hub which carries the connecting means for connecting the injection needle to an injection apparatus is usually moulded from a suitable thermoplastic material. The connecting means can be any kind of suitable means to establish a connection between the injection pen and the injection needle.

**"Cartridge"** is the term used to describe the container containing the drug. Cartridges are usually made from glass but could also be moulded from any suitable polymer. A cartridge or ampoule is preferably sealed at one end by a pierceable membrane which can be pierced e.g. by the non-patient end of a needle cannula. The opposite end is typically closed by a plunger or piston made from rubber or a suitable polymer. The plunger or piston can be slidable moved inside the cartridge. The space between the pierceable membrane and the movable plunger holds the drug which is pressed out as the plunger decreased the volume of the space holding the drug. However, any kind of container - rigid or flexible - can be used to contain the drug.

All references, including publications, patent applications, and patents, cited herein are incorporated by reference in their entirety and to the same extent as if each reference were individually and specifically indicated to be incorporated by reference and were set forth in its entirety herein.
All headings and sub-headings are used herein for convenience only and should not be constructed as limiting the invention in any way.
The use of any and all examples, or exemplary language (e.g. such as) provided herein, is intended merely to better illuminate the invention and does not pose a limitation on the scope of the invention unless otherwise claimed. No language in the specification should be construed as indicating any non-claimed element as essential to the practice of the invention. The citation and incorporation of patent documents herein is done for convenience only and does not reflect any view of the validity, patentability, and/or enforceability of such patent documents.
This invention includes all modifications and equivalents of the subject matter recited in the claims appended hereto as permitted by applicable law.

### BRIEF DESCRIPTION OF THE DRAWINGS:

The invention will be explained more fully below in connection with a preferred embodiment and with reference to the drawings in which:
- Figure 1A-E: show the sequence of the working of the dose injection mechanism in one embodiment.
- Figure 2A-E: show the sequence of the working of the dose injection mechanism in a different embodiment.

The figures are schematic and simplified for clarity, and they just show details, which are essential to the understanding of the invention, while other details are left out. Throughout, the same reference numerals are used for identical or corresponding parts.

### DETAILED DESCRIPTION OF EMBODIMENT:

When in the following terms as "upper" and "lower", "right" and "left", "horizontal" and "vertical", "clockwise" and "counter clockwise" or similar relative expressions are used, these only refer to the appended figures and not to an actual situation of use. The shown figures are schematic representations for which reason the configuration of the different structures as well as their relative dimensions are intended to serve illustrative purposes only.

In that context it may be convenient to define that the term "distal end" in the appended figures is meant to refer to the end of the injection device which usually carries the injection needle whereas the term "proximal end" is meant to refer to the opposite end pointing away from the injection needle and usually carrying the injection button.

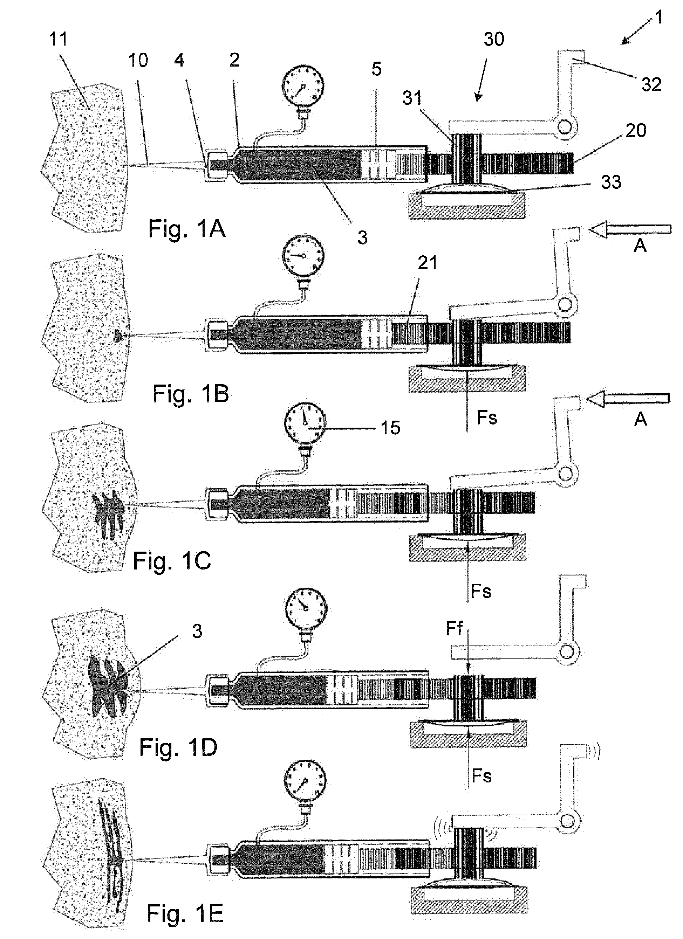

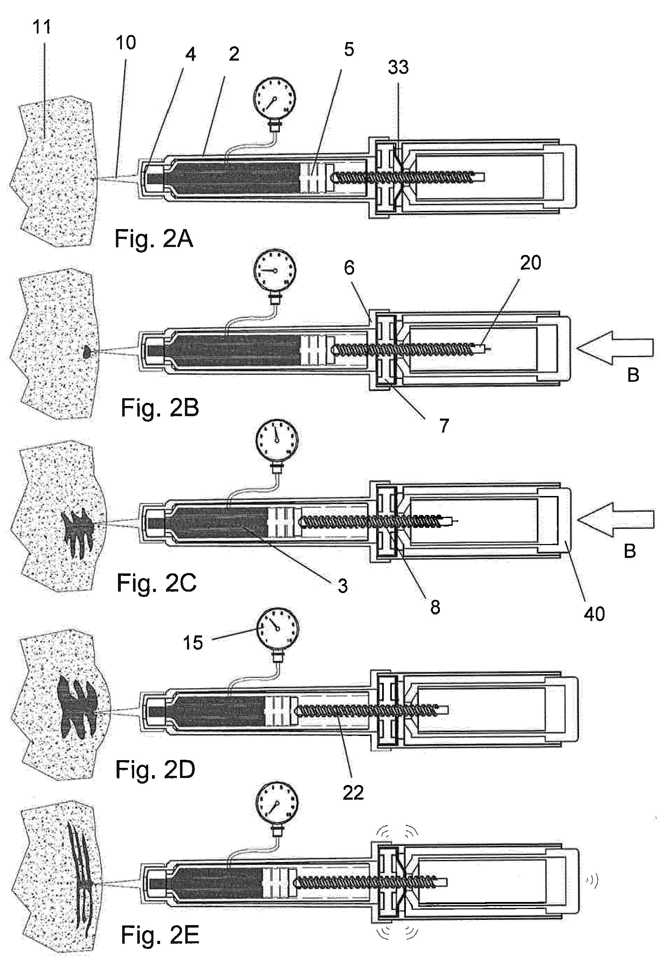

Figure 1 and figure 2 discloses an injection device 1 comprising a cartridge 2 containing a liquid drug 3 to be injected into an object 11 through an injection needle 10. The cartridge 2 is closed at one end by a pierceable membrane 4 through which the injection needle 10 can be brought into liquid communication with the liquid drug 3 in the cartridge 2. In the opposite, proximal end, the cartridge 2 is closed by a plunger 5 which when moved forward presses the liquid drug 3 out through the injection needle 10 and into the object 11.

To move the plunger 5 in the distal direction a piston rod 20 is provided which piston rod 20 is moved by a dose injection mechanism.

In figure 1 A-E, the pressure surveillance system 30 comprises a gearwheel 31 which engages similar teeth 21 provided radially on the piston rod 20.

When a user activates the injection mechanism to inject a dose, a lever 32 moves (arrow A) the gear wheel 31 radially in relation to the piston rod 20. The radial movement of the gear wheel 31 tightens a spring 33 which there after applies a radial force Fₛ on the gear wheel 31. At the same time, the piston rod 20 and the plunger 5 moves in the distal direction which builds up a pressure inside the cartridge 2 as indicated with a pressure measuring apparatus 15 in figure 1C. The applied pressure makes the liquid drug 3 flow from the cartridge 2 and into the object 11 through the injection needle 10 as depictured in figure 1B-1D. At the same time the pressure which has built up inside the cartridge 2 results in an axial force applied proximally onto the plunger 5 and hence on the piston rod 20.

When the user no longer activates the injection mechanism as depictured in figure 1D, the lever 32 tilts back to its initial position. However, the pressure inside the cartridge 2 continue to apply an axial force on the plunger 5 and the piston rod 20 in the proximal direction which pressure creates a frictional force F_{f} between the teeth 21 of piston rod 20 and the gear wheel 31 which prevents the spring 33 from releasing its force Fₛ and thereby move the gear wheel 31 in the radial direction. It assumed that the wheel 31 is coupled to a one-way mechanism such that it can only rotate in one rotational direction.

When the pressure has dropped below a certain threshold value as indicated in figure 1E, the liquid drug 3 will stop flowing through the injection needle 10. Further, as no pressure, or only little pressure, is present inside the cartridge 2 as indicated in the pressure measuring apparatus 15 in figure 1E, the backward force exerted on the piston rod 20 will also approach zero. This will decrease the frictional force F_{f} between the piston rod 20 and the gear wheel 31 and thus allow the spring 33 to move the gearwheel 31 in a radial direction under influence of the force Fₛ as Fₛ grow larger than F_{f}.

This radial movement of the gear wheel 31 will provide a tactile and/or audible signal to the user as indicated in figure 1E, thus signalling to the user that the injection needle 10 can be removed from the body.

Figure 2A-E discloses an embodiment based on a rotatable piston rod 20. This piston rod 20 is provided with an external helical thread 22. The injection device 1 comprises a housing 6 which is further provided with a nut member 7 having an internal thread 8 mating the external thread 22 of the piston rod 20. Whenever the piston rod 20 is rotated it will also move axially due to threaded interface 8, 22.

A dose injection mechanism is provided which comprises an injection button 40 which the user can press in order to expel a dose of liquid drug as illustrated by the arrow B in figure 2B and 2C.

The dose injection mechanism further comprises a spring 33, which is operated from its relaxed state to its compressed state when the user activates the injection button 40. Figure 2A illustrates the spring 33 in its relaxed state, and figure 2B illustrates the spring 33 in its tensed state. The spring 33 is preferably a disc or plate spring.

When the user activates the injection button 40, the piston rod 20 and the plunger 5 is moved forward inside the cartridge 2 and a pressure is build up inside the cartridge 2 as illustrated by the pressure measuring apparatus 15. This pressure generates an axial force acting on the plunger 5 and the piston rod 20 in the proximal direction.

As the nut member 7 is displaceable a minor distance axially in the housing 6, this axial force will press the nut member 7 in the axial direction and thus jam the spring 33 in its tensed position as illustrated in figure 2B.

As the liquid drug flow from the cartridge 2, the pressure inside the cartridge 2 will decrease. When the pressure falls below a certain threshold value, the axial force on the nut member 7 and on the spring 33 will no longer be sufficient to secure the spring 33 in its tensed position, the spring will thus return to its relaxed state. This changing from tensed state to relaxed state will occur abrupt and thus provide a tactile and/or audible signal to the user that the injection needle 10 can be removed from the object.

Some preferred embodiments have been shown in the foregoing, but it should be stressed that the invention is not limited to these, but may be embodied in other ways within the subject matter defined in the following claims.

## Claims

1. A medical delivery apparatus comprising
A hollow container (2) containing a liquid drug (3), which container (2) is closed at its distal end by a pierceable membrane (4) and open at its proximal end,
A plunger (5) mounted in the container (2) for forward axial movement from the proximal end towards the distal end,
A piston rod (20) for moving the plunger (5) forward,
A dose injection mechanism which can be activated to move the piston rod (20) and the plunger (5) forward towards the distal end and by which forward movement of the plunger (5) a pressure is build up in the liquid drug (3) contained in the container (2),
An injection needle (20) insertable through the membrane (4) to allow to the liquid drug (3) to escape from the container (2) whereby the pressure in the liquid drug (3) contained in the container (2) decreases,
A resilient means (33) which can be activated to operate from a relaxed state to a tensed state upon activation of the dose injection mechanism,
**characterised in that**,
the resilient means (33) are being operated to its tensed state when the pressure is build up in the liquid drug (3) by forward movement of the piston rod (20) and the plunger (5) and which resilient means (33) is secured in its tensed state as long as the pressure inside the container are above a predetermined threshold value,
and which resilient means (33) operates back to its relaxed state when the pressure in the liquid drug (3) decreases below the predetermined threshold value,
and which relaxation of the resilient means (33) provides an audible and/or tactile indication to the user that the pressure in the liquid drug (3) contained in the container (2) is below the predetermined threshold value.

2. A medical delivery apparatus according to claim 1, wherein, the pressure build up inside the container (2) applies an axial force on the plunger (5) and on the piston rod (20).

3. A medical delivery apparatus according to claim 2, wherein, an element of the dose injection mechanism secures the resilient member (33) in its tensed position when the force is applied.

4. A medical delivery apparatus according to claim 3, wherein, the element is a radial movable gear wheel (31).

5. A medical delivery apparatus according to claim 3, wherein, the element is an axial movable nut member (8).

6. A medical delivery apparatus according to any of the previous claims, wherein, the resilient member (33) is a dish spring.

## Patentansprüche

1. Medizinische Verabreichungsvorrichtung, umfassend:
einen hohlen Behälter (2), der ein flüssiges Medikament (3) enthält, wobei der Behälter (2) an seinem distalen Ende mit einer durchstechbaren Membran (4) verschlossen und an seinem proximalen Ende offen ist,
einen Stößel (5), der für eine Axialbewegung nach vorne vom proximalen Ende zum distalen Ende im Behälter (2) montiert ist,
eine Kolbenstange (20), um den Stößel (5) nach vorne zu bewegen,
einen Dosisinjektionsmechanismus, der aktiviert werden kann, um die Kolbenstange (20) und den Stößel (5) nach vorne zum distalen Ende hin zu bewegen, wobei durch diese Vorwärtsbewegung des Stößels (5) ein Druck in dem im Behälter (2) enthaltenen flüssigen Medikament (3) aufgebaut wird,
eine Injektionsnadel (20), die durch die Membran (4) einführbar ist, damit das flüssige Medikament (3) aus dem Behälter (2) entweichen kann, wodurch der Druck in dem im Behälter (2) enthaltenen flüssigen Medikament (3) abnimmt,
ein federndes Mittel (33), das aktiviert werden kann, damit es bei Aktivierung des Dosisinjektionsmechanismus von einem entspannten Zustand zu einem gespannten Zustand wirkt,
**dadurch gekennzeichnet, dass**
das federnde Mittel (33) in seinen gespannten Zustand gelangt, wenn der Druck durch Vorwärtsbewegung der Kolbenstange (20) und des Stößels (5) im flüssigen Medikament (3) aufgebaut wird, und das federnde Mittel (33) so lange in seinem gespannten Zustand befestigt ist, wie der Druck im Behälter über einem vorbestimmten Schwellenwert liegt,
und das federnde Mittel (33) zurück in seinen entspannten Zustand gelangt, wenn der Druck in dem flüssigen Medikament (3) unter einen vorbestimmten Schwellenwert fällt,
und die Entspannung des federnden Mittels (33) eine hörbare und/oder haptische Anzeige gegenüber dem Benutzer dahingehend bereitstellt, dass der Druck in dem im Behälter (2) enthaltenen flüssigen Medikament (3) unter dem vorbestimmten Schwellenwert liegt.

2. Medizinische Verabreichungsvorrichtung nach Anspruch 1, wobei der Druckaufbau in dem Behälter (2) eine Axialkraft auf den Stößel (5) und auf die Kolbenstange (20) ausübt.

3. Medizinische Verabreichungsvorrichtung nach Anspruch 2, wobei ein Element des Dosisinjektionsmechanismus das federnde Mittel (33) in seiner gespannten Position befestigt, wenn die Kraft ausgeübt wird.

4. Medizinische Verabreichungsvorrichtung nach Anspruch 3, wobei das Element ein radial bewegliches Zahnrad (31) ist.

5. Medizinische Verabreichungsvorrichtung nach Anspruch 3, wobei das Element ein axial bewegliches Mutterelement (8) ist.

6. Medizinische Verabreichungsvorrichtung nach einem der vorhergehenden Ansprüche, wobei das federnde Mittel (33) eine Tellerfeder ist.

## Revendications

1. Appareil d'administration médical comprenant
un réceptacle creux (2) contenant un médicament liquide (3), ledit réceptacle (2) étant fermé au niveau de son extrémité distale par une membrane perçable (4) et ouvert au niveau de son extrémité proximale,
un piston (5) monté dans le réceptacle (2) en vue d'un mouvement axial vers l'avant de l'extrémité proximale vers l'extrémité distale,
une tige de piston (20) servant à déplacer le piston (5) vers l'avant,
un mécanisme d'injection de dose qui peut être activé afin de déplacer la tige de piston (20) et le piston (5) vers l'avant en direction de l'extrémité distale, et ledit mouvement vers l'avant du piston (5) entraînant une augmentation de la pression dans le médicament liquide (3) se trouvant dans le réceptacle (2),
une aiguille d'injection (20) qui peut être insérée à travers la membrane (4) afin de permettre au médicament liquide (3) de s'échapper du réceptacle (2), ceci se traduisant par une baisse de la pression dans le médicament liquide (3) se trouvant dans le réceptacle (2),
un moyen élastique (33) qui peut être activé afin de passer d'un état relâché à un état tendu lors de l'activation du mécanisme d'injection de dose,
**caractérisé en ce que**
le moyen élastique (33) est mis dans son état tendu lorsque la pression augmente dans le médicament liquide (3) du fait du mouvement vers l'avant de la tige de piston (20) et du piston (5) et ledit moyen élastique (33) étant fixé dans son état tendu tant que la pression à l'intérieur du réceptacle est supérieure à une valeur seuil prédéterminée,
et ledit moyen élastique (33) repassant à son état relâché lorsque la pression dans le médicament liquide (3) baisse en dessous de la valeur seuil prédéterminée,
et ledit relâchement du moyen élastique (33) fournissant une indication audible et/ou tactile à l'utilisateur que la pression dans le médicament liquide (3) se trouvant dans le réceptacle (2) est inférieure à la valeur seuil prédéterminée.

2. Appareil d'administration médical selon la revendication 1, dans lequel l'augmentation de la pression à l'intérieur du réceptacle (2) applique une force axiale sur le piston (5) et sur la tige de piston (20).

3. Appareil d'administration médical selon la revendication 2, dans lequel un élément du mécanisme d'injection de dose fixe l'élément élastique (33) dans sa position tendue lorsque la force est appliquée.

4. Appareil d'administration médical selon la revendication 3, dans lequel l'élément est une roue dentée mobile radialement (31).

5. Appareil d'administration médical selon la revendication 3, dans lequel l'élément est un élément formant écrou mobile axialement (8).

6. Appareil d'administration médical selon l'une quelconque des revendications précédentes, dans lequel l'élément élastique (33) est un ressort à disque.
